Europäisches Patentamt

European Patent Office

Office européen des brevets

(19)

(11) Publication number: **0 131 552**
**A2**

(12) # EUROPEAN PATENT APPLICATION

(21) Application number: **84830195.8**

(22) Date of filing: **27.06.84**

(51) Int. Cl.⁴: **C 07 K 15/14**
**G 01 N 33/53**

(30) Priority: **29.06.83 IT 4859383**

(43) Date of publication of application:
**16.01.85 Bulletin 85/3**

(84) Designated Contracting States:
**AT BE CH DE FR GB LI NL SE**

(71) Applicant: **ISTITUTO FARMACOLOGICO SERONO SpA**
**Via Ludovisi 35**
**I-00187 Roma(IT)**

(72) Inventor: **Sartori, Claudia**
**via Tronto, 18**
**I-00198-Rome(IT)**

(74) Representative: **Santostefano, Alfredo**
**c/o Studio Ing. C. Gregorj S.r.l. Via Sallustiana1/A**
**I-00187 Roma(IT)**

(54) Purified seminal antigenic fraction, process for its preparation and use of said fraction for the dosage of the spermagglutinating antibodies.

(57) A purified seminal antigenic fraction, consisting of five glycoproteins being characterized, each of them, by a specific apparent molecular weight and by a specific isoelectric point, is obtained from seminal plasma and/or sperm coating proteins by means of immunoaffinity chromatography.

EP 0 131 552 A2

The seminal antigenic fraction is useful for the research and the dosage of spermagglutinating antibodies.

The existence of couple infertility due to the presence, in the female organism, of spermagglutinating antibodies is well known. Said antibodies are generally indicated by the term of "antispermatozoon" antibodies, because they interfere with the fertilizing power of spermatozoa in the female genital tract. The antibody titre being considered interfering with fertility has been indicated by 1:32. More recently, and basing upon larger series of cases, Shulman has mentioned the titre of 1:4 as significantly interfering with the progress of the spermatozoa in the female genital tract.

The type of agglutination which is more frequently found in female sera which are positive in this sense i.e. a head-to head agglutination, has induced to think that the antigens being responsible of such a case are residing in the material which covers the spermatozoon (the cellular coat). The verification of such a hypothesis requires the availability of purified preparation of the nemaspermic coat: on the other hand, the coat adheres to the surface of the spermatozoon in such a way, that it does not consent the detaching of it using the common washinf methods:moreover said washings are

**0131552**

atle to damage the cellular structures being the most important from the antigenic point of view, i.e. the plasmatic membrane and the acrosome. Such a type of damaging effect does not consent to verify the exclusive presence ot the coat proteins in a preparations after sperm washing. In order to obtain from one side coat-free spermatozoa from the other side, some pure preparation of coating material, a method has been found, which consents to obtain, from viable spermatozoa, the coat release without interfering with the ultrastructure of the cells.

At this point, it has been possible to show that the female sera having spermagglutinating properties did completeley lose said properties when they were tested with coat-free spermatozoa: t.is observation has induced to state that the antibodies, being present in the positive female sera, are in effect, directed against the coat antigens. Some specific analyses have permitted, later, to show the compositional identity of the coat with the seminal plasma: with the light of said results the observation that the spermagglutinating female sera do react immunologically with the seminal plasma coming from vasectomized subjects, therefore being deprived of components having a nemaspermic derivation, takes a more significant value.

The results of the studies made up to now in order to identify the specific antigens of the human seminal fluid, have given results being often inconsistent between each other, and anyway, not being able to be interpreted in a univocal way. The most significant case with reference to the above is represented by the scaferrin. This molecule, which is similar if not identical to the molecule of the lactoferrin, has been considered the most important specific antigen of the human nemaspermic coat, and several researches have indicated its importance in this respect. Recently, it has been demonstrated that the antisera produced against the seminal lactoferrin (scaferrin = sperm coating antigene & ferrin) are unable to induce any specific agglutination of the spermatozoa. Similar results have been obtained when one did operate with other seminal antigens.

At the basis of the instant invention there is the individuation and the isolation in a reproducible manner, of the antigenic fraction of the seminal plasma (and therefore also of the spermatozoal coat).

Said antigenic fraction, specifically reacting with spermagglutinating antibodies, is the primary subject of the instant invention, and is characterized by the fact of being

constituted by five molecular substances of a glycoproteic
nature, having apparent molecular weights of 170,000: 120,000:
90,000: 50,000 and 25,000 when they are submitted to the
gel-electrophoretic analysis, having isoelectric points of
4.3: 5.0: 5.2: 6.0 and 6.4 when they are submitted to the
isoelectric focusing analysis, and having glycidid residues
consisting of mannose and/or glucose.

It has been found, moreover, that each one of the five
glycoproteins composing the antigenic fraction is able to react
with spermagglutinating antibodies. The individual
glycoproteins, each one being characterized by a specific
apparent molecular weight when submitted to the
gel-electrophoretic analysis, are therefore further subjects
of the instant invention.

At the isoelectric focusing analysis, five different
isoelectric points have been individuated: they represent an
equal number of characterizing values of the individual
glycoproteins and of the antigene fraction as a whole.

The process followed in order to individuate and to separate,
in a reproducible manner, the antigenic fraction according to
the invention, may be schematically represented in the
following process steps :

0131552

- isolation, from seminal plasma coming from individuals which have been vasectomized since 4 months at least, of the glycoproteins by means of affinity chromatography on Concanavalin-A-Sepharose :

- some verification of the presence of the antigens according to the instant invention, in the glycoproteic fraction, by means of the absorption of the fraction with reference spermagglutinating female sera :

- purification from any spermagglutinating female serum, of the total Ig**G**, and conjugation of the latter ones to a chromatographic support :

- immuno-affinity chromatography of the seminal glycoproteins on the conjugated female IgG :

- collection from the column: 1) of the not retained fraction and 2) of the specifically retained fraction :

- immunologic verification of the antigenic activity in the fraction retained by the IgG :

- qualitative analysis of the material obtained :

- production of antisera directed against the isolated antigenes.

As stating material in the above schematically described process, were seminal fluids collected from individuals of different blood groups, which have submitted themselves to vasectomy since at least 4 months.

The samples have been kept at room temperature into sterile plastic reservoirs until a complete fluidification did occur and then, after a microscopical check with phase contrast on dark field were submitted to a bacteriological screening and were stored at - 30° until the time of use.

On separate aliquots of said samples, the dosage of fructose and of the DNA was performed in order to ascertain the homogeneity of the collected material, and to exclude any cellular contamination.

The isolation of the seminal glycoproteins from the seminal plasma has been performed by means of an affinity chromatography on Concanavalin-A (Con-A), which permitted to obtain the glycoproteic fraction through a specific elution. To this purpose, the seminal material has been brought to ice temperature and immediately dialyzed at 2 C, in presence of PMSF (Phenyl-Methane Sulphonyl Fluoride) 1mM as a specific proteases inhibitor, against a Tris-HCl 5 mM buffer with NaCl 0.5M addition and later submitted to an affinity chromatography on Concanavalin-A being conjugated to CN Br Separose 4B (Pharmacia) (column size = 2×40 cm). The fractions not retained by the ConA and the fraction eluted by means of alpha-methyl-mannose 0.5M have been collected, dialyzed and lyophilized.

For the preparation of the spermagglutinating female sera, and for the purification, from the above mentioned ones, of the total IgG, some samples of venous blood have been collected from partner women of unfertile couples wherein the post-coital (P.C.T.) tests did show some agglutination of the spermatozoa at the cervical level, and wherein the research of the spermagglutinating antibodies in the blood serum had given some values which were considered of importance (1:32 at least). Several serum samples have been pooled and submitted to a salting-out by means of saturated ammonium sulphate, with the purpose to obtain the precipitation of the IgG. The fraction obtained as above, which was not pure in the gel-electrophoretic analysis, has been submitted to a chromatography on DEAE-Cellulose, thus obtaining a preparation of IgG which resulted to be pure according to the gel-electrophoretic analysis. The chromatographic fraction has been dialyzed, against cold distilled water for 24 hours in presence of PMSF 1 mM and lyophilized. The spermagglutination tests have shown the activity of the above isolated IgG.

The conjugation of the antibodies with the chromatographic support, and the performing of the affinity chromatography have been made using CN Br-Sepharose 4B (Pharmacia) according to the manufacturer's instructions.

Therefore, these methods are not described in detail. In the experience being at the basis of the instant invention, conjugated samples of about 4 mg of IgG per ml of resin have been obtained and used.

## EXAMPLE 1

The seminal plasma coming from individuals who have been vasectomized since 4 months at least has been collected, tested and conserved, following the above mentioned technique.

The plasma has been defrosted, and later has been submitted to a chromatography on Con-A, according to the above described general process.

The glycoproteic fraction which has been eluted by the Concanaval-A was dialyzed against distilled water, lyophilized, dissolved in a Tris-HCl 5 mM, pH buffer solution containing NaCl 0,5 M, and chromatographed (10 ml/hour at 4°C) on the female IgG which were prepared according to the above indicated method. The fraction, which was not retained in the column, has been collected, dialyzed against distilled water, lyophilzed and stored separately. The eluted fraction from the immunocomplexes which were formed in the column (elution with NaSCN 3M in water) has been collected, dialyzed against distilled water and lyophilized.

The glycoproteic fraction brought to zero the titre of the reference sera, whereas no variations of this titre have been obtained after adsortion of the fraction excluded by the Con-A. The eluted fraction (antigenic fraction) thus obtained has been tested, according to the following methods :

a) macroscopic agglutination test (according to Schulman): in a few words, this test provides the incubation of the antigen with serial dilutions of a spermagglutinating serum having a known titre, followed by a further titration, made after having separated the immune compounds which were formed. The titration is made by making use of spermatozoa taken from donor seminal samples :

b) microscopic agglutination test (T.A.T. = Tray Agglutination) Test): this test provides the incubation of aliquots of normal and viable spermatozoa, with serial dilutions of a spermagglutinating reference serum. The microscopic observation allowes the agglutinating titre of the serum to be measured: the same research repeated after incubation of the reference

**0131552**

serum with the antigen permits to ascertain whether the adsorption has been performed or not.

The same antigenic fraction has been analyzed in electrophoresis on a polyacrylamide gel by applying standard (Coomassie blue) and specific staining for the glycoproteins (Schiff). The electrophoreses were performed both on rod gels and slabs. Moreover, an isoelectric focusing (pH 3.5-9.0) of the antigens eluted from the female IgG has been performed. The antigenic fraction has caused a setting to zero of the reference serums titres, whereas no variations of this titre has been obtained after adsorption of the fraction which was not retained by the IgG. The gel electrophoretic analysis has shown the presence of five bands, also resulting after isoelectric focusing. Table 1 shows the apparent molecular weights of the glycoproteins after isolation from the IgG. The same table reports also the iso-electric points of the above mentioned glycoproteins.

TABLE I

| Molecular weights | | Isoelectric points |
|---|---|---|
| band n.1 | 170,000 | 4.3 |
| band n.2 | 120,000 | 5.0 |
| band n.3 | 90,000 | 5.2 |
| band n.4 | 50,000 | 6.0 |
| band n.5 | 25,000 | 6.4 |

The quantity of antigens obtained from the immunoaffinity chromatography on the female spermagglutinating IgG has been sufficient to perform basic research on the composition (SDS-PAGE, isoelectrofocusing), immunological properties, immunodiffusion; however, it has not permitted to prepare some specific antisera in animals.

In order to have available a larger quantity of specific antigens, an original method has been found; which is a further object of the instant invention. In a few words, this original method comprises the following operating steps :

1) production of antisera against the glycoproteins which are not retained by the IgG (the non retained percentage represents the greatest part of the glycoproteins, as one can see from table 2):

2) isoltation and conjugation of the IgG obtained to CNBr-Sepharose :

3) affinity immunochroatography of the total seminal glycoproteins on the conjugated IgG and collection, as a not retained fraction, of the specific antigens.

TABLE II

Output of the isolation of the glycoproteins

|  | (mg) | (%) |
|---|---|---|
| Total proteins | 300 | 100 |
| Fraction excluded by ConA | 180 | 60 |
| Fraction eluted from ConA | 120 | 40 |

Output of the isolation of the specific antigens

|  | (mg) | (%) |
|---|---|---|
| Total proteins | 50 | 100 |
| Fraction excluded by IgG | 40 | 80 |
| Fraction eluted from IgG | 10 | 20 |

EXAMPLE 2

The seminal plasma coming from vasectomized individuals, who have been operated since 4 months at least, has been collected, tested and stored as described in the Example 1.

The plasma was defrosted and thereafter submitted to a chromatography on Con-A according to the above described general process.

The glycoproteic fraction, after elution from the Con-A has been submitted to affinity chromatography on female IgG, as described in Example 1.

The fraction which was not retained by the chromatographic column has been used as an antigene source for the immunization of adult male albino rabbits weighing, as an average, 2,5 kg in accordance with the following protocol :

First dose: antigen 1 mg per ml of physiological solution plus 1 ml of Freund's complete adjuvant administered in several intradermal injections (0.2 ml per injection).

Second dose at a distance of 7 days from the first one : same preparation as for the first one, but using the Freund's incomplete coadjuvant.

Bloodletting (marginal vein of the ear) after 7 days from the second dose.

Subsequent doses, if any (as a function of the antibody titre obtained) each 15 days in the same way as for the first dose.

The final serum obtained was additioned with 1:1000 sodium methiolate, subdivided into fractions (5 ml each) and frozen at -30°C.

The IgG have been obtained from the final serum, by applying the general method as described in the introductory part of

this text. The antibody fraction has been employed as an immunoadsorbent material in the original process which is described in the following example.

### EXAMPLE 3

The glycoproteic fraction, obtained from seminal plasma, submitted to affinity chromatography on Con-A, in accordance with the process described in the above examples 1 and 2, has been submitted to an affinity chromatography on the IgG obtained as described in the example 2, after conjugation to CN Br-Sepharose in accordance with known procedures.

The not retained (excluded) fraction, from the chromatographic column, submitted to the same tests as the antigenic fraction eluted from the immune compounds with female IgG as described in the example 1, shows the perfect identity with the latter, according to the results of Table 3.

TABLE III

Immunochromatography on spermagglutinating female IgGs.

|                                                        | Excluded | Eluted   |
| ------------------------------------------------------ | -------- | -------- |
| Number of bands in SDS-PAGE                            | over 10  | 5        |
| Appararent molecular weights                           | -------- | 170,000  |
|                                                        |          | 120,000  |
|                                                        |          | 90,000   |
|                                                        |          | 50,000   |
|                                                        |          | 25,000   |
| Immunodiffusion against sperm-agglutinating female serum |  −      | +        |
| Antiserum, from total blood                            | +        | −        |
| Anti-excluded by spermaggluti-nating female IgG        | +        | −        |
| Activity adsorption, female positive reference serum   | −        | +        |

0131552

### Immunochromatography on rabbit's IgG

|  | Excluded | Eluted |
|---|---|---|
| Number of bands in SDS-PAGE | 5 | over 10 |
| Apparent molecular weights | 170,000 | ----- |
|  | 120,000 |  |
|  | 90,000 |  |
|  | 50,000 |  |
|  | 25,000 |  |
| Immunodiffusion against spermagglutinating female serum | + | - |
| Antiserum, from total blood | - | + |
| Anti-excluded by spermagglutinating female IgG | - | + |
| Activity adsorption, female positive reference serum | + | - |

The use of the IgGs which have been isolted from antisera obtained in the animals immunized with the seminal glycoproteins deprived of any specific antigen, permits to produce considerable quantities of specific antigens, as a fraction which is not retained by the immunoadsorbent material. This process has the additional advantage in that it supplies the specific antigens as a not retained fraction in immunocomplexes, with the result, that it is possible to isolate these antigens without using chemical cleaving systems for the immunocomplexes, thus assuring about the native state of the antigenic preparation.

The process of the invention is able to isolate, from the human total seminal plasma, considerable quantities of antigens which are specific for the female "spermagglutinins". This result makes possible some developments of a valuable practical interest such as the research and the dosage of spermagglutinating antibodies in the biological fluids (blood, semen, cervical mucos) in "in vitro" tests, without using fresh seminal specimens of an optimum quality and without the need to have any qualified personnel available, in order to perform and to evaluate the above mentioned tests.

The following example describes the use of seminal antigens which were purified according to the invention, for the research and the dosage of spermagglutinating antibodies in the blood serum of a woman, who was the partner of an infertile couple.

EXAMPLE 4

The research has been performed basing upon the results of a study of the seminal fluid (which, however, was quite normal), of the hormonal situation of the partners (normal) and of two subsequent post coital tests (PCT), which had shown the presence of agglutinated spermatozoa in the cervical mucus. The research and the dosage of the specific antibodies has been performed in accordance with the Kibrick's method (Gelatin Agglutination Test, G.A.T.), with the Friberg's method (Tray Agglutination Test, T.A.T.) and finally by using the preparation of purified seminal antigens according to the method of the instant invention (Seminal Purified Antigen Method, S.P.A.M.).

G.A.T. Method

a) material being the source of antibodies: blood serum serially diluted from 1/'4 to 1/1024:

b) material being the source of antigens: seminal fluid collected from a healthy and fertile donor. The sample is submitted to a sequence of centrifugations and suspensions of the spermatozoa (final concentration: $40 \cdot 10^6$ spermatozoa per ml).

c) performance: a 10% gelatine solution in a buffer solution (PBS or Baker's) is prepared: the solution is kept at 37°C. Equal volumes of spermatozoa suspension and of gelatin are pooled, and the preparation is kept at 37°C. To 0.2-0.3 ml of each serum dilution an equal volume of spermatozoa suspension in gelatine is added, by operating at 37°C.

Each serum preparation-spermatozoa gelatine is passed to a capillary tube.

The various capillary tubes are kept in a vertical position at the temperature of 37°C after having closed the bottom ends of them.

The reading is performed after one and two hours of incubation. The agglutination appears in form of white aggregates. The results obtained with the application of the above described method to theserum sample which is submitted to the investigation, are reported in Table IV.

T.A.T. Method

a) material being the source of antibodies: the same as the G.A.T. method.

b) material being the source of antigens: the same as for the G.A.T. method.

c) performance: 5 microliters of each serum dilution are passed to a microchamber tray by using a Hamilton syringe under a drop of mineral oil. The microsyringe must be carefully washed after each passage. One microliter of spermatozoa suspension is added, operating as described above, to each serum drop.

Then incubation follows at room temperature for 4 hours.

By using an inverted microscope, each microwell is read: the agglutination type and the agglutinating titre of the serum under investigation are determined.

The results obtained with the above described T.A.T. method are reported in Table IV.

Method with purified seminal antigenes (S.P.A.M.)

a) material being the source of antibodies: the same as for both above described methods:

b) material being the source of antigens: a lyophilized powder being equal to 1 mg of purified seminal antigens which have been obtained with the method of the invention.

c) performance: solution of the antigens in one ml of phosphate buffer solution, or of physiological solution. Addition of 0.1 -0.2 of each dilution of the serum being under research, to an equal volume of antigens solution.

Incubation at 37°C for about 30 minutes and direct visualization (nefelometry), or otherwise after centrifugation, of the occured formation of immune complexes.

The results obtained with the S.P.A.M. method are reported in Table IV.

0131552

## TABLE IV

| Method | Tested sample | Antibody titre |
|--------|---------------|----------------|
| G.A.T. | Female serum | 1:128 |
| T.A.T. | " | 1:64 |
| S.P.A.M. | " | 1:256 |

The results obtained show the following advantages of the S.P.A.M. method in comparison with the G.A.T. and T.A.T.

methods:

- the sensibility is higher:

- the incubation time is reduced by 3/4:

- no particular manual operations are required: no sophisticated or uncommon instruments are required:

- any interference and cause of a specific results, caused by

seminal elements such as bacteria, virus and mycoplasma, is excluded:

- the use of human seminal material of an oprtium quality and in fresh state is not required:

- the stability of the lyophilized seminal antigen is

considerable.

- 23 -

0131552

## CLAIMS

1) Seminal antigenic fraction which reacts in a specific manner with spermagglutinating antibodies, characterized in that it is composed of five molecular compounds of a glycoproteic nature, which present apparent molecular weights of 170,000: 120,000: 90,000: 50,000 and 25,000 when submitted to gel-electrophoretic analysis, which present isoelectric points 4.3: 5.0: 5.2: 6.0 and 6.4 when submitted to isoelectric focusing analysis and which present glycide residues of mannose and/or glucose.

2) Glycoprotein component of the antigenic fraction in accordance with Claim 1, characterized in that it presents an apparent molecular weight of 170,000.

3) Glycoprotein component of the antigenic fraction in accordance with Claim 1, characterized in that it presents an apparent molecular weight of 120,000.

4) Glycoprotein component of the antigenic fraction in accordance with Claim 1, characterized in that it presents an apparent molecular weight of 90,000.

5) Glycoprotein component of the antigenic fraction in accordance with Claim 1, characterized in that it presents an apparent molecular weight of 50,000.

0131552

6) Glycoprotein component of the antigenic fraction in accordance with Claim 1, characterized in that it presents an apparent molecular weight of 25,000.

7) Antibody fraction obtained from animals immunized with the glycoproteic fraction excluded from the immunoaffinity chromatography of the total of the glycoproteins from seminal plasma and/or coating proteins of the spermatozoa, performed by using as immunoadsorbent the purified IgG from spermagglutinating female sera.

8) Process for preparing the antibody fraction in accordance with Claim 7, comprising the operational steps of :

a) submitting seminal plasma and/or coating proteins of the spermatozoa, to an affinity chromatography of Con-A in order to isolate the glycoproteic fraction by means of a specific elution with alpha-methyl-mannose 0.5 M:

b) submitting the thus obtained glycoproteic fraction to an immunoaffinity chromatography by using, as immunoadsorbent material, the purified IgG from spermagglutinating female sera:

c) collecting the fraction which was excluded in (b):

d) producing specific antisera by immunizing animals with the above mentioned excluded fraction.

9) Process for preparing the antigenic fraction according to Claim 1, comprising the operational steps of :

a) submitting some seminal plasma and/or coating proteins of the spermatozoa to an affinity chromatography on Con-A, in order to isolate the glycoproteic fraction by means of a specific elution:

b) submitting the thus obtained glycoproteic fraction to an immunoaffinity chromatography by using, as an immunoadsorbent material, the antibody fraction in accordance with Claim 7, in order to obtain the desired antigenic fraction as a fraction excluded from the column.

10) Use of the antigenic seminal fraction according to Claim 1, or otherwise of any one of the glycoproteins in accordance with Claims 2 to 6, for the research and the dosage of spermagglutinating antibodies.